# EUROPÄISCHE PATENTSCHRIFT

(11) **EP 0 620 856 B1**
(45) Veröffentlichungstag und Bekanntmachung des Hinweises auf die Patenterteilung: **03.09.1997**
(21) Anmeldenummer: 93900043.6
(22) Anmeldetag: 14.12.1992
(51) Int. Cl.: C12P 7/42

(54) **HYDROXYLIERUNG VON ALKYLCARBONSÄUREN MIT HILFE VON PILZEN**
HYDROXYLATION OF ALKYL CARBOXYLIC ACIDS USING FUNGI
HYDROXYLATION D'ACIDES ALKYLCARBOXYLIQUES AU MOYEN DE CHAMPIGNONS

(30) Priorität: 24.12.1991 DE 4142943
(43) Veröffentlichungstag der Anmeldung: 26.10.1994
(73) Patentinhaber: BASF Aktiengesellschaft, 67063 Ludwigshafen (DE)
(72) Erfinder: LADNER, Wolfgang, D-6701 Fussgoenheim (DE); STAUDENMAIER, Horst, Ralf, D-6703 Limburgerhof (DE); HAUER, Bernhard, D-6701 Fussgeonheim (DE); MUELLER, Ursula, D-6701 Fussgoenheim (DE); PRESSLER, Uwe, D-6701 Altrip (DE); MEYER, Joachim, D-6701 Maxdorf (DE); SIEGEL, Hardo, D-6720 Speyer (DE)
(86) Internationale Anmeldenummer: EP9202891
(87) Internationale Veröffentlichungsnummer: WO9313214

(56) Entgegenhaltungen:
- WO-A-90/11362
- DE-A- 1 905 648
- CHEMICAL ABSTRACTS, vol. 86, no. 13, 28. März 1977, Columbus, OH (US); K. KOHMOTO et al., Seite 228, Nr. 85840x
- CHEMICAL ABSTRACTS, vol. 67, no. 9, 28. August 1967, Columbus, OH (US); S.M. BOCKS, Seite 4808, Nr. 51378v

## Beschreibung

Die Erfindung betrifft ein Verfahren zur Hydroxilierung mit Hilfe von Mikroorganismen.

Es ist bekannt, daß Mikroorganismen aromatische Verbindungen hydroxilieren können. Dabei entstehen in der Regel bishydroxilierte Verbindungen oder Gemische verschiedener Regioisomerer (Biochem J. 65, 682 (1957)). Weiter ist bekannt, daß man 2-Phenoxipropionsäure regioselektiv zu 2-(4-Hydroxiphenoxi)-propionsäure oxidieren kann (WO 90/11362).

Es wurde ein Verfahren zur Hydroxilierung von Verbindungen der Formel I gefunden 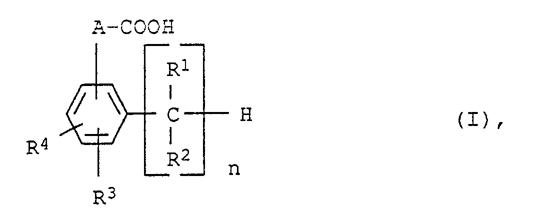 worin
- n: 0 oder 1,
- A: eine direkte Bindung, eine C₁-C₄-Alkylen- oder eine C₁-C₄-Alkylenoxigruppe,
- R¹: ein Wasserstoffatom, eine C₁-C₈-Alkyl, C₁-C₄-Alkoxi- oder C₁-C₄-Alkylthiogruppe,
- R²: ein Wasserstoffatom oder eine C₁-C₈-Alkylgruppe, die gegebenenfalls durch ein Halogenatom oder eine C₁-C₄-Alkoxigruppe substituiert ist,
- R³: ein Halogenatom oder eine C₁-C₄-Alkyl-, C₁-C₄-Alkoxi-, Nitro-, Nitril-, Aminocarbonyl oder C₁-C₄-Alkoxicarbonylgruppe sowie eine Phenoxi- oder Naphthoxigruppe, die durch gegebenenfalls 1 bis 2 Halogenatome, Hydroxi-, C₁-C₄-Alkyl- oder C₁-C₄-Alkoxigruppen substituiert sein können und
- R⁴: ein Wasserstoff- oder Halogenatom oder eine C₁-C₄-Alkyl- oder C₁-C₄-Alkoxigruppe
darstellen oder deren Salzen, dadurch gekennzeichnet, daß man die Verbindungen der Formel I oder deren Salze unter aeroben Bedingungen in Gegenwart eines Pilzes der Gattung Beauveria hydroxiliert.

Das Verfahren eignet sich besonders zur Hydroxilierung solcher Verbindungen der Formel I, in der n 0 oder 1, R¹ eine Methyl-, Ethyl- oder Isopropylgruppe, R² ein Wasserstoffatom oder eine Methylgruppe, R³ ein Halogenatom (insbesondere Fluor, Chlor oder Brom), eine Methyl-, Methoxi-, Ethoxioder Phenoxigruppe und R⁴ ein Wasserstoffatom, ein Halogenatom (insbesondere Fluor, Chlor oder Brom) oder eine Methylgruppe bedeuten.

Für das Verfahren eignen sich eine Vielzahl von Pilzen. Brauchbare Pilze lassen sich beispielsweise aus Erdproben, insbesondere humushaltigen Bodenproben, isolieren. Besonders geeignet für die Umsetzung sind Beauveria-Stämme. Diese Pilze können beispielsweise aus Stammsammlungen bezogen werden. Ihre Eignung für die Hydroxilierung läßt sich in einem einfachen Vorversuch ermitteln. Als besonders günstig haben sich die Stämme Beauveria bassiana erwiesen.

Zur Durchführung des erfindungsgemäßen Verfahrens werden geeignete Stämme auf ein die Verbindung I enthaltendes Nährmedium überimpft und darin bei für den jeweiligen Mikroorganismus günstigen Wachstums- bzw. Produktionsbedingungen aerob inkubiert. Die Fermentation erfolgt kontinuierlich oder diskontinuierlich für 1 bis 10 Tage.

Die Zellen des verwendeten Mikroorganismus, die auch in Form von ruhenden, nicht wachsenden Zellen verwendet werden können, läßt man direkt auf das Substrat einwirken. Es können beliebige, bekannte Inkubationsverfahren angewendet werden, besonders bevorzugt sind aber Fermenter in Form von tiefen, belüfteten und gerührten Tanks. Sehr gute Ergebnisse werden durch Inkubation eines flüssigen Nährmediums erhalten.

Geeignet sind Nährmedien, die Kohlenstoffquellen, Stickstoffquellen, anorganische Salze und gegebenenfalls geringe Mengen an Spurenelementen und Vitaminen enthalten. Als Stickstoffquellen können anorganische bzw. organische Stickstoffverbindungen oder Materialien, die diese Verbindungen enthalten, verwendet werden. Beispiele sind Ammoniumsalze, Nitrate, Maisquellwasser, Bierhefeautolysat, Sojabohnenmehl, Weizengluten, Hefeextract, Hefe, Harnstoff und Kartoffelprotein. Als Kohlenstoffquellen können beispielsweise Zucker wie Glucose, Polyole wie Glycerin oder Fette wie Sojaöl verwendet werden.

Beispiele für anorganische Salze sind die Salze von Calcium, Magnesium, Mangan, Kalium, Zink, Kupfer, Eisen und anderen Metallen. Als Anion der Salze ist besonders das Phosphation zu nennen. Gegebenenfalls werden dem Nährmedium Wachstumsfaktoren zugesetzt, wie z.B. Biotin, Riboflavin oder andere Vitamine.

Das Mischungsverhältnis der genannten Nährstoffe hängt von der Art der Fermentation ab und wird im Einzelfall festgelegt.

Im allgemeinen eignen sich zur Durchführung des erfindungsgemäßen Verfahrens Konzentrationen an Verbindung I von etwa 1 bis 100 g/l, bevorzugt sind Konzentrationen von etwa 5 bis 50 g/l.

Die Züchtungsbedingungen werden so festgelegt, daß die bestmöglichen Ausbeuten erreicht werden. Bevorzugte Züchtungstemperaturen liegen bei 20°C bis 40°C. Besonders vorteilhaft sind Temperaturen zwischen 25°C und 30°C. Vorzugsweise wird der pH-Wert in einem Bereich von 3 bis 9 festgehalten. Besonders vorteilhaft sind pH-Werte zwischen 4 und 7. Im allgemeinen ist eine Inkubationsdauer von 15 bis 100 Stunden ausreichend. Innerhalb dieser Zeit reichert sich die maximale Menge des gewünschten Produkts im Medium an. Die Säure wird vorzugsweise in Form eines Salzes, z.B. des Natriumsalzes, zugegeben. Die Säure kann dem Nährmedium am Anfang auf einmal, nach erfolgtem Wachstum oder während der Züchtung in mehreren Portionen oder kontinuierlich zugegeben werden.

Bevorzugt ist der Einsatz der Verbindung I als freie Säure, man kann aber auch deren Salze einsetzen. Bevorzugte Salze sind Alkali- und Erdalkalisalze, beispielsweise die Na-, K-, Li-Salze.

Das neue Verfahren eignet sich sowohl zur Oxidation von racemischen Verbindungen I als auch von deren Antipoden. Beim erfindungsgemäßen Verfahren bleibt in der Regel das Asymmetriezentrum unangetastet. Das neue Verfahren stellt somit einen einfachen und kostengünstigeren Weg zur selektiven Herstellung von Hydroxi-Derivaten der Verbindungen I dar.

Für den Eintritt der Hydroxigruppe in die Verbindung I gelten folgende Regeln:
- Wird ein aromatischer Ring hydroxiliert, dann tritt der Sauerstoff in die 4-Position ein.
- Von zwei aromatischen Ringen wird der elektronenreichere hydroxiliert.
- Befindet sich eine oxidierbare Seitenkette am Aromaten, dann können prinzipiell Kernhydroxilierung und Seitenkettenoxidation stattfinden. Welche Oxidation bevorzugt abläuft, ist von der Natur der Seitenkette (z.B. sterischer Hinderung) und von der Position am Aromaten abhängig:
   2-Position führt bevorzugt zur Kernhydroxilierung,
   3-Position führt bevorzugt zur Seitenkettenoxidation
   4-Position führt ausschließlich zur Seitenkettenoxidation.
   Eine 2-Methylgruppe wird noch zu ca. 35 % oxidiert.
   Eine 2-Ethylgruppe wird nicht mehr oxidiert.
- Ist die Seitenkette eine n-Alkylgruppe, dann findet immer die Oxidation an Benzylkohlenstoff statt.
- Sterisch anspruchsvolle Gruppen wie die Isopropylgruppe werden endständig oxidiert. Die Oxidation ist ganz überwiegend enantioselektiv. Die absolute Konfiguration des neu entstandenen Chiralitätszentrums ist noch unbekannt.
- Als Oxidationsprodukte der Seitenkette entstehen immer die entsprechenden Alkohole.
- Sind mehrere Seitengruppen oxidierbar, dann wird immer nur eine Gruppe zum Alkohol oxidiert. Besonders bevorzugt sind hier die Seitenkette an der 4-Position. Ist dort kein Substituent vorhanden, erfolgt die Oxidation der Gruppe in 3- bzw. 5-Position.

Die hydroxilierten Verbindungen I stellen wertvolle Zwischenprodukte zur Herstellung von Pflanzenschutzmitteln (US-A-4 750 931) und Pharmawirkstoffen dar.

Die folgenden Beispiele erläutern die Erfindung:

### Beispiel

Es wurde folgendes Nährmedium verwendet:

| Medium A | |
|---|---|
| 50 g/l | Glucose |
| 10 g/l | Hefeextrakt |
| 0,5 g/l | Magnesiumsulfat-7-hydrat |
| 1,5 g/l | Kaliumdihydrogenphosphat |
| 3,6 g/l | Dikaliumhydrogenphosphat |
| 3 g/l | Carbopol® 946 (Carboxivinylpolymer mit extrem hohem Molekulargewicht |
| 2 µg/l | Eisen-(II)sulfat-1-hydrat |
| 10 µg/l | Zink(II)sulfat-4-hydrat |
| 300 µg/l | Borsäure |
| 200 µg/l | Cobalt(II) chlorid-6-hydrat |
| 10 µg/l | Kupfer(II) chlorid-2-hydrat |
| 20 µg/l | Nickel (II) chlorid-6-hydrat |
| 30 µg/l | Natriummolybdat-2-hydrat |

Der pH-Wert wurde mit 5 N Natronlauge auf 6,8 eingestellt. Glucose und Phosphate wurden jeweils getrennt bei 121°C für 10 Minuten autoklaviert. Die (R)-2-(Phenoxi-)propionsäure wurde mit wenig 5 N Natronlauge in Wasser gelöst und sterilfiltriert. Das restliche Medium wurde bei 121°C für 10 Minuten autoklaviert.

Jeweils 100 ml des sterilen Mediums wurden in sterile 500 ml Erlenmeyerkolben gefüllt, die mit einem sterilen Watteverschluß versehen waren. Eine Öse Sporen des Pilzstammes Beauveria bassiana ATCC 7159 wurde in einen Kolben eingeimpft. Der Kolben wurde dann bei 28°C und 250 UpM für drei Tage schüttelnde inkubiert. Jeweils 3 dieser Kulturbrühen dienten als Vorkultur für eine Fermentation im 10 l-Maßstab.

In Fermentern mit 10 1 sterilem Medium (Zusammensetzung wie in Beispiel 1) wurden jeweils 10 g/l der in der Tabelle 1 aufgeführten Verbindungen als sterilfiltrierte Na-Salz-Lösung zugesetzt und mit 300 ml der nach Beispiel 1 gewonnenen Vorkultur beimpft. Die Fermentation erfolgte bei 28 bis 29°C unter Rührung mit 400 bis 600 rpm und einer Begasungsrate von 0,5 bis 1,5 vvm. Während der Fermentation wurde gegebenenfalls sterile Glucoselösung (50 w/v) in Mengen von bis zu 50 g/l zugesetzt. Der Verlauf der Umsetzung wurde gaschromatographisch verfolgt. Dazu wurden 1000 µl Fermentationsbrühe entnommen und mit 100 µl konzentrierter Salzsäure und 800 µl Essigsäureethylester versetzt und 15 s kräftig durchmischt. 700 ml der organischen Phase wurden vorsichtig abgenommen und bei 50°C unter einem sanften Stickstoffstrom im Probegläschen eingedampft. Der Rückstand wurde in 70 µl Essigsäureethylester gelöst und quantitativ in ein Probegläschen für die Gaschromatographie überführt. Hierzu wurden 30 µl N-Methyl-N-trimethylsilyltrifluoracetamid gegeben. Die Probe wurde dann gaschromatographisch untersucht.

Nach beendeter Fermentation wurde mit konzentrierter Schwefelsäure auf pH 2 angesäuert und die Produkte durch Extraktion mit Methyl-t-butylether, Trocknen mit Na₂SO₄, Eindampfen und gegebenenfalls durch Kristallisation isoliert. Die Identifikation der Umsetzungsprodukte erfolgte mittels ¹H-NMR- und ¹³C-NMR-Spektroskopie. Die Ergebnisse sind in der Tabelle zusammengefaßt.

Die Ausbeuten lagen bei 60 bis 95 %.

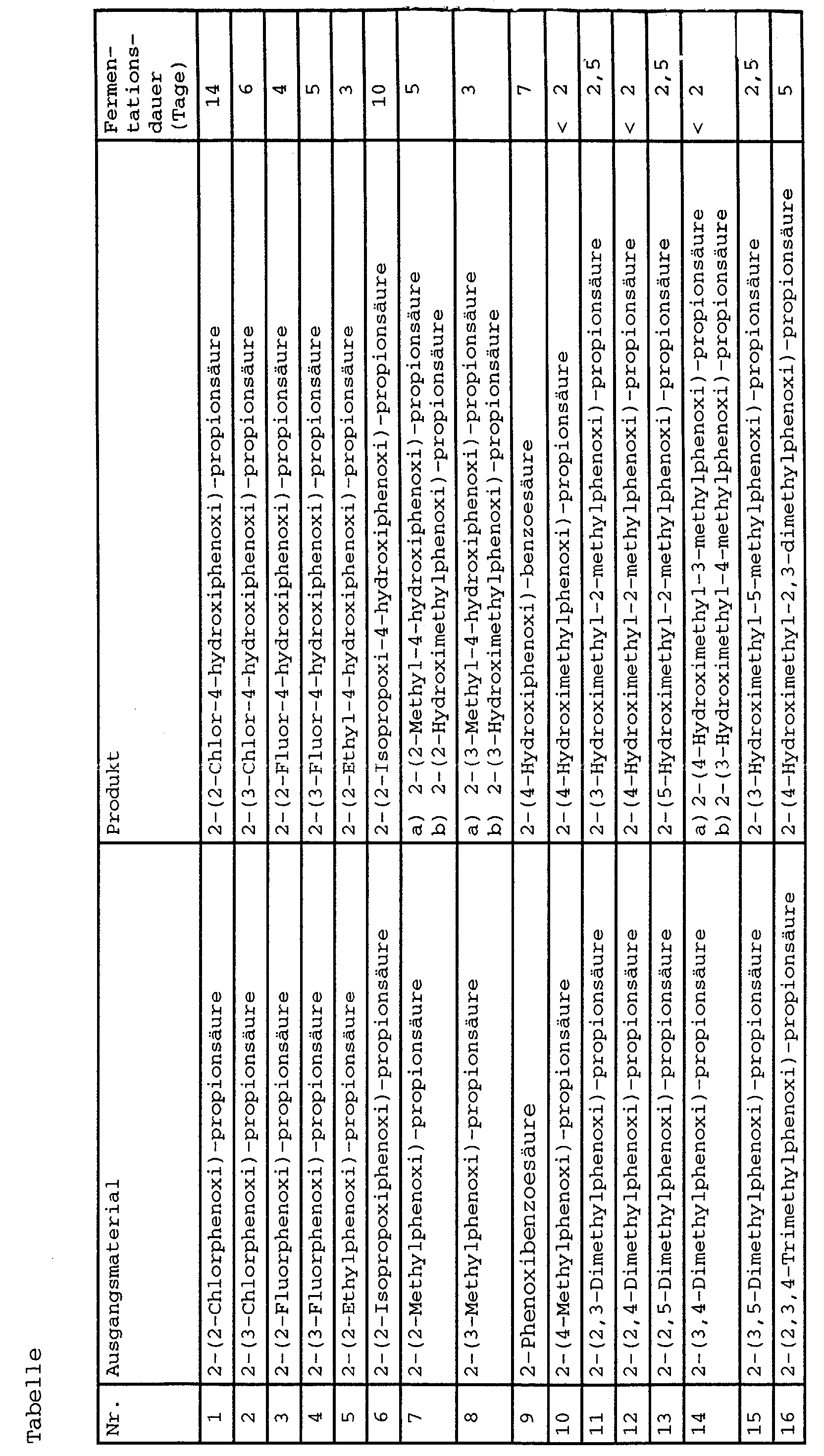

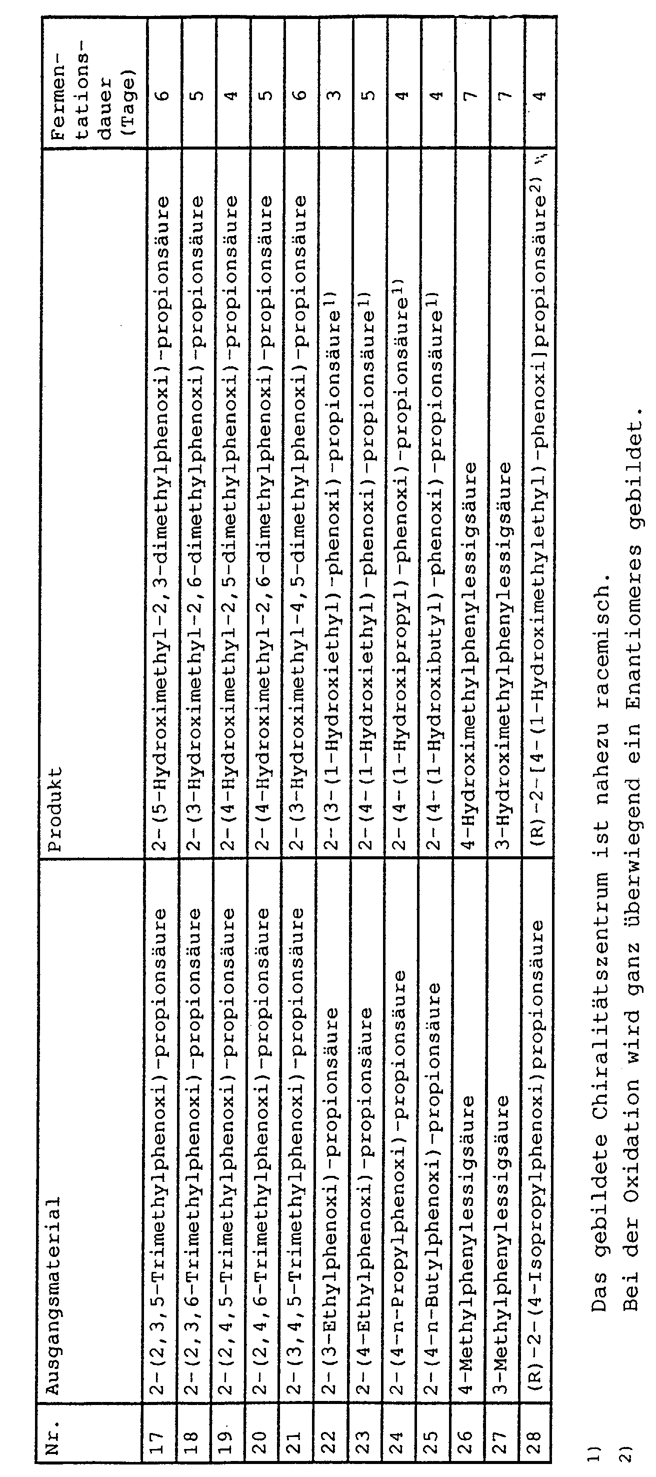

## Patentansprüche

1. Verfahren zur Hydroxilierung von Verbindungen der Formel I 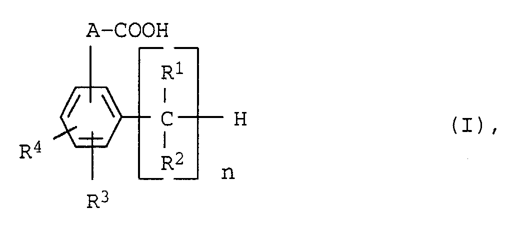 worin
n 0 oder 1,
A eine direkte Bindung, eine C₁-C₄-Alkylen- oder eine C₁-C₄-Alkylenoxigruppe,
R¹ ein Wasserstoffatom, eine C₁-C₈-Alkyl, C₁-C₄-Alkoxi- oder C₁-C₄-Alkylthiogruppe,
R² ein Wasserstoffatom oder eine C₁-C₈-Alkylgruppe, die gegebenenfalls durch ein Halogenatom oder eine C₁-C₄-Alkoxigruppe substituiert ist,
R³ ein Halogenatom oder eine C₁-C₄-Alkyl-, C₁-C₄-Alkoxi-, Nitro-, Nitril-, Aminocarbonyl oder C₁-C₄-Alkoxicarbonylgruppe sowie eine Phenoxi- oder Naphthoxigruppe, die durch gegebenenfalls 1 bis 2 Halogenatome, Hydroxi-, C₁-C₄-Alkyl- oder C₁-C₄-Alkoxigruppen substituiert sein können und
R⁴ ein Wasserstoff- oder Halogenatom oder eine C₁-C₄-Alkyl- oder C₁-C₄-Alkoxigruppe
darstellen oder deren Salzen, dadurch gekennzeichnet, daß man die Verbindungen der Formel I oder deren Salze unter aeroben Bedingungen in Gegenwart eines Pilzes der Gattung Beauveria hydroxiliert.

## Claims

1. A process for hydroxylating compounds of the formula I 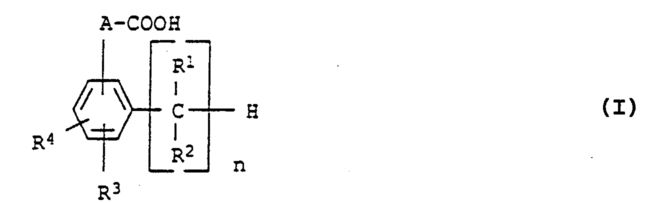 where
n is 0 or 1,
A is a bond, C₁-C₄-alkylene or C₁-C₄-alkyleneoxy,
R¹ is hydrogen, C₁-C₈-alkyl, C₁-C₄-alkoxy or C₁-C₄-alkylthio,
R² is hydrogen or C₁-C₈-alkyl which is unsubstituted or substituted by a halogen or a C₁-C₄-alkoxy,
R³ is halogen, C₁-C₄-alkyl, C₁-C₄-alkoxy, nitro, cyano, carbamoyl or C₁-C₄-alkoxycarbonyl, and phenoxy or naphthoxy which is unsubstituted or substituted by 1 or 2 halogens, hydroxyl, C₁-C₄-alkyl or C₁-C₄-alkoxy groups, and
R⁴ is hydrogen, halogen, C₁-C₄-alkyl or C₁-C₄-alkoxy
or the salts thereof, which comprises hydroxylating the compounds of the formula I or the salts thereof under aerobic conditions in the presence of a fungus of the genus Beauveria.

## Revendications

1. Procédé d'hydroxylation de composés de la formule I 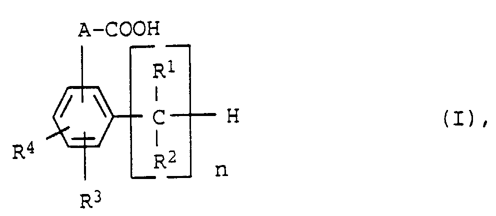 dans laquelle
n est égal à 0 ou à 1,
A représente une liaison directe, un groupe alkylène en C₁ à C₄, ou alkylèneoxy en C₁ à C₄,
R¹ représente un atome d'hydrogène ou un radical alkyle en C₁ à C₈, alcoxy en C₁ à C₄, ou alkylthio en C₁ à C₄,
R² représente un atome d'hydrogène ou un groupe alkyle en C₁ à C₈, qui peut éventuellement être substitué par un atome d'halogène ou un radical alcoxy en C₁ à C₄,
R³ représente un radical alkyle en C₁ à C₄, alcoxy en C₁ à C₄, nitro, nitrile, aminocarbonyle, ou alcoxy(C₁ à C₄)carbonyle, comme aussi un radical phénoxy ou naphtoxy, qui peuvent éventuellement être substitués par un ou deux atomes d'halogènes, radicaux hydroxyle, alkyle en C₁ à C₄, ou alcoxy en C₁ à C₄ et
R⁴ représente un atome d'hydrogène ou un atome d'halogène, ou un radical alkyle en C₁ à C₄, ou alcoxy en C₁ à C₄,
et de leurs sels, caractérisé en ce que l'on hydroxyle les composés de la formule I ou leurs sels dans des conditions aérobies et en présence d'un champignon du genre Beauveria.
